# EUROPÄISCHE PATENTSCHRIFT

(11) **EP 0 374 946 B1**
(45) Veröffentlichungstag und Bekanntmachung des Hinweises auf die Patenterteilung: **23.03.1994**
(21) Anmeldenummer: 89123766.1
(22) Anmeldetag: 22.12.1989
(51) Int. Cl.: G01N 33/53, G01N 33/68

(54) **Verfahren zum Nachweis von kohlenhydrathaltigen Verbindungen**
Method for the detection of carbohydrate-containing compounds
Procédé de détection des composés contenant des glucides

(30) Priorität: 23.12.1988 DE 3843598; 11.01.1989 DE 3900639
(43) Veröffentlichungstag der Anmeldung: 27.06.1990
(73) Patentinhaber: BOEHRINGER MANNHEIM GMBH, 68298 Mannheim (DE)
(72) Erfinder: Haselbeck, Anton, Dr., D-8120 Weilheim (DE); Hösel, Wolfgang, Dr., D-8132 Tutzing (DE); von der Eltz, Herbert, Dr., D-8120 Weilheim (DE); Schickaneder, Edith, D-8000 München 71 (DE)
(74) Vertreter: Huber, Bernhard, Dipl.-Chem.

(56) Entgegenhaltungen:
- EP-A- 0 041 426
- EP-A- 0 149 405
- EP-A- 0 166 623
- EP-A- 0 171 243
- EP-A- 0 218 347
- WO-A-88/00702
- HISTOCHEMICAL JOURNAL, Band 19, 1987; D. VOLZ et al., Seiten 311-318#

## Beschreibung

Die Erfindung betrifft ein Verfahren zum Nachweis von kohlenhydrathaltigen Verbindungen, sowie ein dazu geeignetes Reagenz.

Kohlenhydrathaltige Verbindungen sind weit verbreitet. Neben Glycerinderivaten, wie z.B. Monoglyceriden, die z.B. als Emulgatoren Verwendung finden und in Lebensmitteln vorkommen, sind vor allen Dingen die Zucker allgegenwärtig, hier insbesondere die Pentosen und Hexosen, sowie deren Derivate. Die Zucker kommen im Organismus in vielen Variationen, insbesondere auch in Form von Glycokonjugaten wie z.B. Glycoproteinen vor. Sehr viele Wirkstoffe im Organismus können nur transportiert werden oder sind nur wirksam, wenn sie in glycosilierter Form vorliegen. Es ist daher wünschenswert, derartige Verbindungen sowohl quantitativ als auch qualitativ aus Probelösungen sowie auch auf Gewebsschnitten mit hoher Genauigkeit nachweisen zu können. Weiterhin ist es wichtig, die einzelnen Zucker bzw. OH-Gruppen-haltigen Verbindungen voneinander unterscheiden zu können.

Es sind bereits viele Verfahren bekannt, mit denen kohlenhydrathaltige Verbindungen mit chemischen oder biochemischen Methoden sowohl quantitativ als auch qualitativ nachgewiesen werden können. So ist es beispielsweise bekannt, ein Zuckergemisch chromatographisch oder elektrophoretisch aufzutrennen und anschließend durch Anfärbung die einzelnen Zucker sichtbar zu machen. Geeignet sind beispielsweise die Reaktion mit Phenolschwefelsäure, die Reaktion mit Perjodat und anschliessende Färbung mit Schiff'schem Reagenz oder Silber oder anschließende Dansyl-fluorometrische Bestimmung. Nachteil dieser chemischen Methoden ist es, daß sie entweder nicht sehr spezifisch sind oder aber zu unempfindlich sind. Die empfindlicheren chemischen Methoden haben wiederum den Nachteil, daß sie sehr umständlich durchzuführen sind und sehr störanfällig sind.

Weiterhin war es z.B. aus DE-OS 36 29 194 bekannt, Glycoreste in Konjugaten durch Reaktion mit einem Biotinderivat und anschließende Umsetzung mit einem markierten Streptavidin nachzuweisen. Dieses Verfahren hat jedoch den Nachteil, daß durch einen hohen Background die Methode nur wenig empfindlich ist.

Es war daher Aufgabe der Erfindung, ein Verfahren zur Verfügung zu stellen, mit dem kohlenhydrathaltige Verbindungen empfindlich und spezifisch sowohl qualitativ als auch quantitativ nachgewiesen werden können. Weiterhin sollte ein Verfahren entwickelt werden, das einfach durchzuführen ist und breite Anwendungsmöglichkeiten bietet.

Diese Aufgabe wird gelöst durch ein Verfahren zum Nachweis von kohlenhydrathaltigen Verbindungen, das dadurch gekennzeichnet ist, daß man die nachzuweisende Verbindung mit einer Verbindung (im Weiteren als Konjugat bezeichnet) umsetzt, die eine Gruppierung, die eine spezifische Bindung an den Kohlenhydrat-Teil der nachzuweisenden Substanz ermöglicht, und ein Hapten mit einem Molekulargewicht von 300 - 1200 enthält, anschließend den gebildeten Komplex mit markierten, gegen das Hapten gerichteten Antikörpern in Kontakt bringt und die Markierung in an sich bekannter Weise bestimmt.

Überraschenderweise wurde gefunden, daß mit dem erfindungsgemäßen Verfahren kohlenhydrathaltige Verbindungen sehr empfindlich nachgewiesen werden können. Das Verfahren eignet sich sowohl zur Anfärbung auf Chromatogrammen und Gewebeschnitten, als auch zur quantitativen Auswertung. Obwohl die Bindungskonstante der Biotin/Avidin-Wechselwirkung mit K=10¹⁵ Mol⁻¹ um mindestens den Faktor 10⁵ höher ist als die Bindungskonstante der Wechselwirkung von Haptenen mit den entsprechenden Antikörpern, die im Bereich von K=2x10⁸ Mol⁻¹ bis 7x10⁹ Mol⁻¹ liegen, und außerdem die Biotin/Avidin-Wechselwirkung begünstigt ist, da im Avidinmolekül vier Biotin-Bindungsstellen vorliegen, erlaubt das erfindungsgemäße Verfahren eine Nachweissensitivität, die mindestens so groß ist wie die Nachweissensitivität des Biotin/Avidin-Systems. Ein weiterer überraschender Vorteil ist es, daß bei Verwendung der Haptene und den entsprechenden Antikörpern praktisch kein Background auftritt. Außerdem ist das Verfahren einfach und reproduzierbar durchzuführen.

Das erfindungsgemäße Verfahren ist geeignet zum Nachweis von kohlenhydrathaltigen Verbindungen. Insbesondere geeignet ist es zum Nachweis von Hexosen, Pentosen und Sialinsäure, deren Derivate sowie zuckerhaltigen Substanzen (Glycokonjugate) wie z.B. den Glycoproteinen. Das erfindungsgemäße Verfahren kann in der klinischen, biochemischen und lebensmittelchemischen Diagnostik nun so variiert werden, daß sowohl der Gesamtgehalt an Zuckern, als auch einzelne Verbindungen in einem Gemisch nachgewiesen werden können.

Die Verbindungen können sowohl in Lösung (vorzugsweise mit einem ELISA-Test) als auch gebunden an einen Träger nachgewiesen werden. Das erfindungsgemäße Verfahren ist besonders geeignet für alle Glycokonjugate, die in fixierter Form vorliegen, sei es auf einem Träger oder in einem Gewebe.

Die Glycoproteine können bereits direkt in dem Gel, das zur Auftrennung des Gemisches dient, nachgewiesen werden. Bevorzugt ist es jedoch, das aufgetrennte Gemisch zuerst auf einen Träger zu überführen und anschließend die gewünschte Verbindung nachzuweisen.

Zur Durchführung des erfindungsgemäßen Verfahrens wird zuerst die nachzuweisende Verbindung mit einem Konjugat umgesetzt, das eine Gruppierung, die eine spezifische Bindung an die nachzuweisende Substanz ermöglicht und ein Hapten mit einem Molekulargewicht zwischen 300 und 1200 enthält.

Die Gruppierung wird danach ausgesucht, wie spezifisch der Nachweis sein soll. Geeignet sind einerseits Substanzen, die spezifisch mit der nachzuweisenden Verbindung in unveränderter Form reagieren. Hier kommen vor allem Lectine oder kohlenhydrat-spezifische Antikörper (Biochem.I. 245, (1987) 1-11) in Betracht. Es sind verschiedene Lectine bekannt, die mit einzelnen Zuckerarten spezifisch binden (Ann.Rev.Biochem. 55 (1986) 35-67). Diese Lectine können aus Pflanzen isoliert werden und sind im Handel erhältlich. Verwendet werden können z.B. Peanut-Agglutinin (PNA), das spezifisch mit endständig gebundenen Galactoseresten bindet, Wheat germ-Agglutinin (WGA), das spezifisch für N-Acetylglucosamin ist, Galanthus nivalis Agglutinin (GNA), das spezifisch mit endständig gebundenen Mannoseresten bindet, Sambucus nigra Agglutinin (SNA), das spezifisch ist für 2-6 gebundene Sialinsäuren sowie Maackia amurensis Lectin (MAL), das spezifisch 2-3 gebundene Sialinsäuren bindet. Der Fachmann kann also leicht das für den jeweiligen Zweck geeignete Lectin herausfinden. Als Antikörper sind Antikörper und deren Fragmente geeignet, die mit der nachzuweisenden Substanz spezifisch reagieren. Eine weitere Möglichkeit besteht darin, die nachzuweisende Verbindung spezifisch zu oxidieren und dann als Gruppierung eine mit dem entstehenden spezifisch oxidierten Rest, in der Regel eine Aldehyd- oder Carboxylgruppe, reagierende funktionelle Gruppe zu verwenden.

Die Vorbehandlung kann dabei in an sich bekannter Weise durchgeführt werden. Bevorzugt wird der Kohlenhydrat-Teil der zu bestimmenden Verbindung mit einem spezifischen Enzym oder mit Perjodat oxidiert. Dabei entstehen jeweils Aldehydgruppen. Als damit bindefähige funktionelle Gruppen sind Amine und Hydrazine geeignet.

In einer besonderen Ausführungsform wird daher die nachzuweisende Verbindung mit einem Konjugat umgesetzt, das einen Amin- oder Hydrazidanteil enthält. Im Falle des Amins werden die dabei entstehenden Schiff'schen Basen besonders bevorzugt anschließend an diese Umsetzung noch dehydriert, um die instabile Aldiminbindung in eine stabile Aminbindung zu überführen.

Die Oxidation des Kohlenhydrat-Anteils erfolgt unter an sich bekannten Bedingungen. Wird ein spezifisches Enzym (z.B. Galactose-Oxidase oder Glucose-Oxidase) eingesetzt, so werden die für die Enzymaktivität günstigsten Bedingungen ausgewählt. Die Bedingungen für die Oxidation mit Perjodsäure sind ebenfalls an sich bekannt. In der Regel wird die Oxidation mit 5 bis 15 mmol/l Perjodat bei Zimmertemperatur ca. 10 bis 30 Minuten durchgeführt. Wenn eine einzelne Verbindung spezifisch nachgewiesen werden soll, werden die Oxidationsbedingungen in an sich bekannter Weise so eingestellt, daß nur die gewünschte Verbindung oxidiert wird. Dabei können je nach zu behandelnder Verbindung auch noch mildere Bedingungen sinnvoll sein. Im Anschluß an die Perjodatoxidation ist es zweckmäßig, noch in der Lösung befindliches, überschüssiges Perjodat durch Zugabe von Bisulfit zu zerstören.

Der zweite Bestandteil des Konjugats ist ein Hapten mit einem Molekulargewicht von 300 - 1200. Dieses Hapten dient dazu, eine Markierung spezifisch zu binden. Geeignet sind beispielsweise Steroide (wie z.B. Digoxin, Digoxigenin, Cortisol, Oestriol, Oestradiol, Theophyllin, Testosterol, Gallensäuren, Progesteron und Aldosteron); kurzkettige Peptide (wie z.B. Argipressin, Oxytocin und Brachykinin); Fluorescein und seine Derivate; T₃, T₄, Aflatoxin, Atrazin, Pflanzenhormone wie z.B. Gibberilline; und Alkaloide (wie z.B. Reserpin und Ajmalicin). Bevorzugt wird ein Hapten verwendet, das nicht endogen in dem Organismus vorkommt, aus dem die jeweilige Probe stammt. Insbesondere werden nicht natürlich vorkommende Haptene wie z.B. anellierte Aromaten verwendet.

Besonders bevorzugt werden als Hapten Digoxin, Digoxigenin, Fluorescein und Derivate sowie Theophyllin eingesetzt.

Das Hapten kann direkt an die Gruppierung, die die spezifische Bindung vermittelt, gebunden sein. Bevorzugt ist das Hapten jedoch über einen Spacer an die Gruppierung gebunden. Der Spacer sollte dabei eine Länge im Bereich von 3 bis 32 Atomen haben. Er ist in der Regel aus Molekülen aufgebaut, die die Atome C, O, S oder/und N enthalten.

In einer bevorzugten Ausführungsform der Erfindung ist das Steroidhapten über einen Spacer von 3 bis 16 Atomen Länge gebunden. Besonders bevorzugt wird für Lectine ein Spacer der Länge 3 bis 9 Atome und für Amine oder Hydrazide ein Spacer der Länge 9 bis 16 Atome verwendet. Bevorzugt werden als Spacer N-Acyl-ε-aminocapronsäuren verwendet. Besonders bevorzugt sind N-Acetyl-ε-aminocapronsäure und N-Succinyl-ε-aminocapronsäure.

Nach Durchführung der ersten Stufe des erfindungsgemäßen Verfahrens bildet sich ein Komplex aus nachzuweisender Verbindung und Konjugat. Wenn nun eine Auftrennung mit Chromatographie, insbesondere Gelchromatographie oder Gelelektrophorese, erfolgen soll, so kann dies entweder vor oder nach der Bildung des Komplexes erfolgen. Dies ist abhängig von der nachzuweisenden Substanz. Das erfindungsgemäß verwendete Konjugat hat ein relativ geringes Molekulargewicht, so daß es eine Auftrennung durch SDS-Gelelektrophorese nicht beeinträchtigt, wenn die nachzuweisende Verbindung eine höhermolekulare Substanz wie beispielsweise ein Glycoprotein ist. In diesem Fall kann daher zuerst die Umsetzung mit dem Konjugat erfolgen und anschließend die Auftrennung in dem Gel. Der Nachweis kann dann direkt im Gel geführt werden. Bevorzugt werden die aufgetrennten Fraktionen durch Blotting auf eine Membran (z.B. Nitrocellulosefilter, Nylon- oder Polyvinylmembran) überführt und dort sichtbar gemacht. Ebenso kann, insbesondere wenn die nachzuweisenden Verbindungen ein relativ niedriges Molekulargewicht haben, zuerst die Auftrennung erfolgen und anschließend die Umsetzung mit dem erfindungsgemäßen Konjugat und der Nachweis der Markierung. So kann z.B. ein Zuckergemisch zuerst chromatographisch aufgetrennt werden und anschließend durch Umsetzung mit dem erfindungsgemäßen Konjugat und dem entsprechenden markierten Antikörper nachgewiesen werden. Die jeweils geeignete Methode ist abhängig von der nachzuweisenden Substanz und kann im speziellen Fall leicht herausgefunden werden.

Der aus nachzuweisender Substanz und Konjugat gebildete Komplex wird anschließend umgesetzt mit einem gegen das Hapten gerichteten markierten Antikörper. Als Antikörper können monoklonale oder polyklonale Antikörper oder deren Fragmente und Derivate verwendet werden. Derartige Antikörper sind dem Fachmann bekannt.

Die Markierung des Antikörpers erfolgt in an sich bekannter Weise. Geeignet sind z.B. die Markierung mit einem Enzym, die radioaktive Markierung, die Markierung mit einer bio- oder chemilumineszenten Verbindung oder einer fluoreszierenden Verbindung oder mit einer Verbindung, die ein solches Signal verursacht. Bevorzugt wird als Markierung ein Enzym verwendet. Besonders bevorzugt wird als Enzym alkalische Phosphatase, Peroxidase oder β-Galactosidase verwendet. Insbesondere bevorzugt bei der Durchführung des erfindungsgemäßen Verfahrens ist als Markierungsenzym alkalische Phosphatase. Die Bestimmung der Markierung erfolgt in an sich bekannter Weise. Wird als Markierung ein Enzym verwendet, so muß zum Nachweis ein Substrat zugegeben werden, das eine nachweisbare Reaktion liefert. Bevorzugt werden hierzu Leukosysteme, insbesondere indigoide Systeme als oxidierbare Verbindungen und Tetrazoliumsalze als Oxidationsmittel verwendet. Besonders geeignet ist ein Redox-System, das aus 5-Brom-4-chlor-3-indolylphosphat (X- phosphat) und Nitroblau-Tetrazolium besteht. Dabei spaltet die alkalische Phosphatase das X-Phosphat, das durch die Abspaltung des Phosphats und Oxidation ein blaues, schwer lösliches Dimer bildet, wobei gleichzeitig die Tetrazoliumverbindung zu einem ebenfalls blauen, schwer löslichen Formazan reduziert wird.

Eine weitere Möglichkeit der Markierung ist die Markierung mit Gold, die besonders für den Nachweis in Gewebeschnitten geeignet ist.

Bevorzugt erfolgt vor der Umsetzung mit dem Anti-Hapten-Antikörper eine Behandlung mit einem Reagenz, das an die reaktionsfähigen Stellen des Trägers sowie der anderen Bestandteile des Gemisches bindet. Geeignet ist hierfür beispielsweise Casein.

Die Nachweisgrenze für einzelne Glycoproteine ist abhängig von der Art des jeweiligen Glycoproteins. Die Empfindlichkeit des erfindungsgemäßen Verfahrens ist jedoch sehr hoch und es können Glycoproteine bis in einem Bereich von 10 ng nachgewiesen werden. Weiterhin können durch das erfindungsgemäße Verfahren verschiedene Zuckerreste unterschieden werden. So können z.B. durch Variation der Oxidationsbedingungen Sialinsäure-haltige Glycoproteine von Asialo-Verbindungen unterschieden werden. Dazu erfolgt die Oxidation mit Perjodat bei sehr milden Bedingungen bei 0°C, bei denen gerade noch die Sialinsäuren, nicht jedoch die Asialo-Verbindungen oxidiert werden. Ebenso können erfindungsgemäß O-glykosidisch gebundene Zucker von N-glykosidisch gebundenen unterschieden werden, indem man das zu untersuchende Gemisch zuerst mit N-glycosidase behandelt und anschließend nach Molekulargewicht auftrennt. Eine positive Zuckerreaktion eines Konjugates deutet dann auf eine O-glykosidische Bindung hin.

Die Anwesenheit von Detergenzien bei der Durchführung des erfindungsgemäßen Verfahrens stört nicht.

Die Erfindung wird durch die folgenden Beispiele erläutert.

### Beispiel 1

### Digoxigenin-O-succinyl-ε-amidocapronsäure-(N-tert.-butyl-oxycarbonyl)-hydrazid

1,40 g (2 mmol) Digoxigenin-O-succinyl-ε-amido-capronsäure-N-hydroxy-succinimidester, der nach dem in DE-A 38 00 644 beschriebenen Verfahren hergestellt wurde, 0,29 g (2,2 mmol) t-Butyloxycarbonyl-hydrazid und 0,29 ml (2,1 mmol) Triethylamin werden 20 Stunden lang bei Raumtemperatur in 8 ml Dioxan unter Feuchtigkeitsausschluß gerührt. Nach Abdestillieren des Lösungsmittels im Vakuum wird zu dem harzigen Rückstand 100 ml Eiswasser gegeben und 4 Stunden stehen gelassen. Dabei kristallisiert das Produkt. Man saugt ab, wäscht mit Eiswasser nach und trocknet über CaCl₂ im Vakuum.
Ausbeute 1,23 g (86 % d. Th.),
MG: 717,9 farbloses Pulver, Fp. 160°C
DC: Kieselgel, Nitromethan/Methanol 8:2, R_{f} = 0,32

### Beispiel 2

### Digoxigenin-O-succinyl-ε-amidocapronsäure-hydrazid-hydrochlorid

1,10 g (1,5 mmol) des tert.-Butyloxycarbonyl-geschützten Produkts werden in 6 ml Trifluoressigsäure gelöst und mit 6 ml CH₂Cl₂ verdünnt. Nach einer Stunde Rühren bei Raumtemperatur werden alle flüchtigen Bestandteile im Vakuum bei 40°C abgezogen und zweimal mit je 5 ml Dioxan azeotropiert. Das dabei erhaltene farblose Harz wird dann in 30 ml 50 % Methanol aufgenommen, filtriert und über 200 ml DEAE-Sephadex/Cl^{⊖} mit 50 % Methanol chromatographiert. Die erste Fraktion liefert nach Lyophilisieren das gewünschte Produkt.
Ausbeute: 0,69 g (70 % d. Th.) farbloses Pulver
DC: Kieselgel, Nitromethan/Methanol 8:2, R_{f} = 0,14
MG: 653,2

### Beispiel 3

### Digoxigenin-3-carboxymethylether-ε-amidocapronsäure-(N-tert.-butyloxycarbonyl)-hydrazid

1,32 g (2 mmol) Digoxigenin-3-carboxymethylether-ε-amidocapronsäure-N-hydroxysuccinimidester (Herstellung gemäß DE-A 38 36 656), 0,29 g (2,2 mmol) t-Butyloxycarbonylhydrazin und 0,29 ml (2,1 mmol) Triethylamin werden, wie im Beispiel 1 beschrieben, umgesetzt und aufgearbeitet.
Ausbeute: 1,17 g (89 % d. Th.)
MG: 691,5 farbloses Pulver

### Beispiel 4

### Digoxigenin-3-carboxymethylether-ε-amidocapronsäurehydrazid-hydrochlorid

1,04 g (1,5 mmol) des vorher erhaltenen tert.-Butyloxycarbonyl-geschützten Produkts werden, wie im Beispiel 2 beschrieben, umgesetzt:
Ausbeute: 0,76 g (81 % d. Th.)
MG: 627,8

### Beispiel 5

### Herstellung von digoxigenyliertem Lectin

1x10⁻³ mmol/l Lectin werden in 10 ml 0,05 mol/l Phosphatpuffer, pH 8,5, gelöst. 4x10⁻³ mmol/l (= 2,8 mg) Digoxygenin-O-succinyl-amidocapronsäure-O-succinimidester (Herstellung nach DE-A 38 00 644) werden in 500 µl Dimethylformamid p.A. gelöst und zur Lectinlösung zugegeben.

Man läßt 4 Stunden bei 25°C unter gelegentlichem Schütteln reagieren. Dann wird die Reaktionslösung bei +4°C gegen jeweils 1 l H₂O, dest. 48 Stunden dialysiert, wobei mindestens zweimal das Wasser erneuert wird.

Die dialysierte Lösung wird lyophilisiert.
Ausbeute: 90 bis 100 %.

### Beispiel 6

### Nachweis von Glycanstrukturen in Gewebeschnitten

Es wird im wesentlichen wie in C.F.A. Culling, R.T. Allison und W.T. Barr, Cellular Pathology Technique, Seiten 219 bis 224, Butterworths London, 4. Auflage 1985 beschrieben, vorgegangen.

Danach werden Paraffinschnitte in Xylol und der absteigenden Alkoholreihe fixiert, in Aqua dest. gespült und für 30 Minuten in einer 1%igen Perjodatlösung oxidiert.

Nach Waschen in Aqua dest. wird der Schnitt, der sich auf einem Objektträger befindet, mit 0,1 mol/l Natriumacetat-Puffer, pH 5,5, 10 mmol/l Digoxigenin-O-succinyl-ε-amidocapronsäure-hydrazid-hydrochlorid (Beispiel 2) oder Digoxigenin-3-carboxymethylether-ε-amidocapronsäure-hydrazid-hydrochlorid (Beispiel 4) überschichtet und für 1 bis 2 Stunden in einer feuchten Kammer zur Reaktion gebracht.

Nach dreimaligem Waschen mit TBS (50 mmol/l Tris HCl pH 7,5, 150 mmol/l NaCl) erfolgt die Inkubation mit dem Antikörper: üblicherweise wird eine 1:100 bis 1:1000 Verdünnung in TBS auf den Schnitt aufgebracht (ca. 50 bis 100 µl) und der Schnitt in einer feuchten Kammer für etwa 30 bis 60 Minuten inkubiert.

Nach dreimaligem Waschen mit TBS erfolgt die alkalische Phosphatase-Reaktion durch Eintauchen der Schnitte in 0,1 mol/l Tris-HCl, pH 9,5, 0,1 mol/l Natriumchlorid, 0,05 mol/l Magnesiumchlorid und 5-Brom-4-chlor-3-indolyl-Phosphat (37,5 µl/10 ml; Stammlösung 50 mg/ml in Dimethylformamid) und Nitrotetrazoliumblau (NBT) (50 µl/10 ml; Stammlösung 75 mg/ml in 70 % Dimethylformamid).

Die Farbentwicklung wird mikroskopisch verfolgt und ist in der Regel nach wenigen Minuten optimal. Zum Abstoppen wird der Schnitt in Aqua dest. gespült und eingebettet.

### Beispiel 7

### Nachweis von Glycoproteinen über blotting auf Nitrocellulosemembranen mit digoxigenylierten Lectinen

Eine Lösung, die Glykoproteine enthält, wird in üblicher Weise elektrophoretisch aufgetrennt über eine SDS-PAGE. Nach der Auftrennung werden die Fraktionen durch blotting auf Nitrocellulosemembran (Schleicher und Schüll BA85) übertragen.

Die Nitrocellulose wird nach dem Transfer proteinspezifisch mit Ponceau S angefärbt. Hierzu wird der Blot 5 Minuten in der Ponceau S Lösung, die nach Vorschrift des Herstellers hergestellt worden ist, inkubiert und anschließend mit Wasser gespült, bis rot gefärbte Banden sichtbar werden. Die Standardproteine werden mit einem Bleistift markiert.

Anschließend wird der Blot 30 Minuten lang in einer Lösung von 0,5 % Casein in TBS (50 mmol/l Tris HCl pH 7,5, 150 mmol/l NaCl) inkubiert, wobei 20 ml des Reagenzes pro 50 bis 100 cm² verwendet werden. Bei dieser Inkubation verschwindet die Ponceau S Farbe wieder. Anschließend wird zweimal für je 10 Minuten mit jeweils 50 ml TBS und einmal mit 50 ml TBS, das jeweils 1 mmol/l MgCl₂, MnCl₂ und CaCl₂ enthält, gewaschen.

Es wird eine Lösung hergestellt, die digoxygeniliertes Lectin (Herstellung nach Beispiel 5) in einer Konzentration von 10 µg/ml in TBS sowie jeweils 1 mmol/l MgCl₂, MnCl₂ und CaCl₂ enthält. Mit ca. 10 ml dieser Lösung wird der Blot eine Stunde bei Raumtemperatur inkubiert. Anschließend wird dreimal jeweils 10 Minuten mit TBS gewaschen.

Zur Markierung der nachzuweisenden Glykokonjugate wird der Blot eine Stunde bei Raumtemperatur mit einer Verdünnung eines Antidigoxigenin-Antikörpers, der mit alkalischer Phosphatase gekuppelt ist, in einer Verdünnung von 1:1000 in TBS inkubiert und anschließend dreimal je 10 Minuten mit TBS gewaschen.

Zur Sichtbarmachung der gebundenen Markierung wird der Blot dann kurz in einer Lösung, die 100 mmol/l Tris-HCl, 100 mmol/l NaCl und 50 mmol/l MgCl₂ enthält und einen pH von 9,5 aufweist, gespült und dann mit einer Lösung inkubiert, die 10 ml einer Lösung enthält, die 100 mmol/l Tris-HCl, 100 mmol/l NaCl und 50 mmol/l MgCl₂ enthält und einen pH von 9,5 aufweist, 37,5 µl X-Phosphat (50 mg/ml in Dimethylformamid) und 50 µl Nitrotetrazoliumblau (NBT) (75 mg/ml in 70 % Dimethylformamid). Die Farbentwicklung tritt nach wenigen Minuten ein. Die Färbelösung wird dann entfernt und der Blot mehrfach mit Wasser gespült. Die nachzuweisende Substanz ist auf dem Blot sehr deutlich sichtbar.

### Beispiel 8

### Glykoprotein-Nachweis über blotting mit Digoxigeninhydraziden

Glykoproteine werden in 0,1 mol/l Natriumacetat-Puffer, pH 5,5 mit Hilfe von 10 mmol/l Natriummetaperjodat für 20 Minuten bei Raumtemperatur und im Dunkeln oxidiert. Überschüssiges Natriummetaperjodat wird durch Zusatz von Natriumbisulfit in einer Konzentration von 20 mmol/l zerstört. Nach 5 Minuten wird Digoxigenin-3-carboxymethylether-ε-amidocapronsäure-hydrazid-hydrochlorid oder Digoxigenin-O-succinyl-ε-amidocapronsäure-hydrazid-hydrochlorid in einer Konzentration von 1 mmol/l zugegeben und die Lösung für eine Stunde bei Raumtemperatur inkubiert. Die so behandelten Glykoproteine werden direkt mittels SDS-Gelelektrophorese aufgetrennt und elektrophoretisch auf Nitrozellulose (z.B. BA85 von Schleicher & Schüll) übertragen. Die Nitrozellulosemembran wird für wenigstens 30 Minuten mit 0,5 % Casein in TBS inkubiert und nach mehrmaligem Waschen in TBS mit einer 1:1000 Verdünnung des Anti-Digoxigenin-Antikörpers, der mit alkalischer Phosphatase konjugiert ist, in 10 ml TBS für eine Stunde bei Raumtemperatur inkubiert. Anschließend wird, wie im Beispiel 7 beschrieben, weiter verfahren.

### Beispiel 9

### Glykoprotein-Nachweis über blotting mit Fluoresceinisothiocyanat (FITC)

Es wird, wie im Beispiel 8 beschrieben, verfahren, wobei anstelle von Digoxigeninhydraziden 1 mmol/l 5-(((2-(carbohydrazino)methyl)thio)acetyl)aminofluorescein (R.P. Hangland, Handbook of Fluorescent Probes and Research Chemicals, Molecular Probes, Inc., USA) zugegeben wird.

### Beispiel 10

### Glycoprotein Nachweis in Mikrotiterplatten (ELISA)

50 µl des nachzuweisenden Glycoproteins (Proteinkonzentration: 0.1 µg/ml bis 0.1 mg/ml in 0.05 mol/l Natriumcarbonat-Puffer pH 9,25) werden für 30 Minuten zur Beladung in einen Napf einer Mikrotiterplatte (z.B. NUNC Mikrowell Platte 96F) gegeben. Es werden 100 µl 10 mmol/l Natriumperjodat in 0.1 mol/l Natriumacetat, pH 5.5 zugegeben und 2 Minuten bei Raumtemperatur im Dunkeln inkubiert. Anschließend wird dreimal mit PBS (0.05 mol/l Kaliumphosphat, pH 6.5, 0.15 mol/l Natriumchlorid) gewaschen.

Es werden 100 µl Digoxigenin-O-succinyl-ε-amidocapronsäure-hydrazid-hydrochlorid oder Digoxigenin-3-carboxymethyl-ether-ε- amidocapronsäure-hydrazid-hydrochlorid-Lösung (1 µmol/l in 0.1 mol/l Natriumacetat, pH 5.5) zugegeben und 60 Minuten bei Raumtemperatur inkubiert. Nach Waschen mit PBS (dreimal) wird mit 0.3 ml Blockierungslösung [0.5% Casein in TBS: 0.05 mol/l Tris-HCl, pH 7.5, 0.15 mol/l Natriumchlorid ] pro Napf für 30 Minuten bei Raumtemp. inkubiert. Anschließend wird dreimal mit TBS, 0.1 % Tween 20 gewaschen.

Es werden pro Napf 50 µl Antikörperlösung (1 µl Anti-Digoxigenin-Antikörper, gekoppelt an Peroxidase (150 U/ml), gelöst in 0.5 ml TBS, 0.1 % Tween 20) für 60 Minuten bei Raumtemp. inkubiert und fünfmal mit TBS, 0.1 % Tween 20 gewaschen. Anschließend werden 1,6 mmol/l ABTS® ( 2,2,-Azino-di-(3-ethylbenzthiazolin-sulfonat)) in 95 mmol/l Phosphat-Citrat-Puffer pH 4,4 mit 3,1 mmol/l Natriumperborat zugegeben, 30 Minuten bei Raumtemperatur inkubiert und die Extinktion bei 405 nm bestimmt.

## Patentansprüche

1. Verfahren zum Nachweis von kohlenhydrathaltigen Verbindungen,
**dadurch gekennzeichnet,**
daß man die nachzuweisende Verbindung mit einer Verbindung umsetzt, die eine Gruppierung, die eine spezifische Bindung an den Kohlenhydrat-Teil der nachzuweisenden Substanz ermöglicht, und ein Hapten mit einem Molekulargewicht von 300 - 1200 enthält, anschließend den gebildeten Komplex mit markierten, gegen das Hapten gerichteten Antikörpern in Kontakt bringt und die Markierung in an sich bekannter Weise bestimmt.

2. Verfahren nach Anspruch 1,
**dadurch gekennzeichnet,**
daß man die nachzuweisende Verbindung vor Umsetzung mit dem Konjugat spezifisch oxidiert.

3. Verfahren nach Anspruch 2, **dadurch gekennzeichnet,** daß die nachzuweisende Substanz ein Zucker ist und daß die Oxidation durch eine für diesen Zucker spezifische Oxidase erfolgt.

4. Verfahren nach Anspruch 2, **dadurch gekennzeichnet,** daß die Oxidation durch Einwirkung von Perjodat erfolgt.

5. Verfahren nach Anspruch 1, **dadurch gekennzeichnet,** daß man als Gruppierung, die eine spezifische Bindung an die nachzuweisende Substanz ermöglicht, ein Lectin verwendet.

6. Verfahren nach einem der Ansprüche 1 bis 4,
**dadurch gekennzeichnet,**
daß man als Gruppierung eine mit Aldehyden spezifisch reagierende funktionelle Gruppe verwendet.

7. Verfahren nach einem der vorhergehenden Ansprüche,
**dadurch gekennzeichnet,**
daß man das Hapten an die die spezifische Bindung vermittelnde Gruppe über einen Spacer der Länge 3 - 32 Atome bindet.

8. Verfahren nach einem der vorhergehenden Ansprüche,
**dadurch gekennzeichnet,**
daß als Hapten Digoxin oder Digoxigenin verwendet wird.

9. Verfahren nach einem der vorhergehenden Ansprüche,
**dadurch gekennzeichnet,**
daß man die nachzuweisende Substanz aus einem Gemisch durch Gelelektrophorese auftrennt, auf eine Membran aufnimmt und darauf qualitativ nachweist.

10. Verfahren nach einem der vorhergehenden Ansprüche,
**dadurch gekennzeichnet,**
daß man zuerst das Hapten über die spezifisch bindende Gruppe an die nachzuweisende Substanz bindet, die in einem Gemisch vorliegt und anschließend das Gemisch auftrennt und die Markierung nachweist.

## Claims

1. Method for the detection of compounds containing carbohydrate,
**wherein**
the compound to be detected is reacted with a compound which contains a group which enables a specific binding to the carbohydrate part of the substance to be detected and contains a hapten with a molecular weight of 300 - 1200, afterwards the complex formed is brought into contact with labelled antibodies directed against the hapten and the label is determined in a known way.

2. Method as claimed in claim 1,
**wherein**
the compound to be detected is specifically oxidized before reaction with the conjugate.

3. Method as claimed in claim 2,
**wherein**
the substance to be detected is a sugar and wherein the oxidation is effected by a specific oxidase for this sugar.

4. Method as claimed in claim 2,
**wherein**
the oxidation is effected by the action of periodate.

5. Method as claimed in claim 1,
**wherein**
a lectin is used as the group which enables a specific binding to the substance to be detected.

6. Method as claimed in one of the claims 1 to 4,
**wherein**
a functional group reacting specifically with aldehydes is used as the group.

7. Method as claimed in one of the previous claims,
**wherein**
the hapten is bound to the group mediating the specific binding via a spacer of 3 - 32 atoms in length.

8. Method as claimed in one of the previous claims,
**wherein**
digoxin or digoxigenin is used as the hapten.

9. Method as claimed in one of the previous claims,
**wherein**
the substance to be detected is separated from a mixture by gel electrophoresis, taken up on a membrane and qualitatively detected thereon.

10. Method as claimed in one of the previous claims,
**wherein**
the hapten is firstly bound via the specific binding group to the substance to be detected which is present in a mixture and afterwards the mixture is separated and the label is detected.

## Revendications

1. Procédé de mise en évidence de composés contenant des glucides, caractérisé en ce que l'on fait réagir le composé qui doit être mis en évidence avec un composé qui contient un groupement qui permet une liaison spécifique avec la partie glucidique de la substance qui doit être mise en évidence, et un haptène d'une masse moléculaire de 300 à 1200, puis l'on met en contact le complexe formé avec des anticorps marqués, dirigés contre l'haptène et on détermine le marquage de manière connue en soi.

2. Procédé selon la revendication 1, caractérisé en ce que l'on oxyde spécifiquement le composé qui doit être mis en évidence avant la réaction avec le conjugué.

3. Procédé selon la revendication 2, caractérisé en ce que la substance qui doit être mise en évidence est un sucre et en ce que l'oxydation est réalisée par une oxydase spécifique de ce sucre.

4. Procédé selon la revendication 2, caractérisé en ce que l'oxydation est réalisée par l'action du periodate.

5. Procédé selon la revendication 1, caractérisé en ce que l'on utilise une lectine comme groupement qui permet une liaison spécifique avec la substance qui doit être mise en évidence.

6. Procédé selon l'une des revendications 1 à 4, caractérisé en ce que l'on utilise comme groupement un groupe fonctionnel qui réagit spécifiquement avec les aldéhydes.

7. Procédé selon l'une des revendications précédentes, caractérisé en ce qu'on lie l'haptène au groupe permettant la liaison spécifique par l'intermédiaire d'un espaceur d'une longueur de 3 à 32 atomes.

8. Procédé selon l'une des revendications précédentes, caractérisé en ce que l'on utilise comme haptène la digoxine ou la digoxigénine.

9. Procédé selon l'une des revendications précédentes, caractérisé en ce que l'on sépare la substance qui doit être mise en évidence à partir d'un mélange par électrophorèse sur gel, on la recueille sur une membrane et on la met en évidence qualitativement sur celle-ci.

10. Procédé selon l'une des revendications précédentes, caractérisé en ce qu'on lie tout d'abord par l'intermédiaire du groupe qui se lie de manière spécifique l'haptène à la substance qui doit être mise en évidence, qui se trouve dans un mélange, puis on sépare le mélange et on met en évidence le marquage.
